# EUROPEAN PATENT APPLICATION

(11) **EP 4 696 293 A1**
(43) Date of publication of application: **18.02.2026**
(21) Application number: 23932320.7
(22) Date of filing: 10.04.2023
(51) Int. Cl.: A61H 1/02, A63B 23/035, A63B 23/12

(54) **REHABILITATION AND MONITORING SYSTEM**

(71) Applicant: Umov SPA, Concepción (CL)
(72) Inventor: SALAZAR CIFUENTES, Pamela, Concepción (CL)
(74) Representative: ABG Intellectual Property Law, S.L.
(86) International application number: PCT/CL2023/050035
(87) International publication number: WO 2024/212017

(57) **Abstract**

The present invention consists of a playful rehabilitation and monitoring and/or evaluation system, compact, transportable, that can be used on any surface, place and/or environment, which has a touchscreen monitor that through a web application displays images, words, patterns to follow, games, etc. that promotes upper limb motor function, performing shoulder abduction and external rotation, elbow flexion and extension, among others, that allows the user to improve the range of motion by providing resistance and assistance to the affected joint or body part, which allows moving in a full range of motion.

## Description

### FIELD OF THE INVENTION

A technological rehabilitation and monitoring system that promotes upper limb motor function by facilitating movements such as shoulder abduction and external rotation, elbow flexion and extension, among others, enabling the user to improve range of motion by providing resistance and assistance to the affected joint or body part, thereby allowing movement through a full range of motion.

### BACKGROUND

Cerebrovascular accident (CVA) is a high-prevalence disease with significant public health impact. Its incidence in Chile is at least 97 per 100,000 inhabitants (60 per 100,000 excluding hemorrhagic cerebrovascular accidents) (1). Ischemic CVA represents a major public health issue in Chile, ranking as the third leading cause of death in the country and generating a substantial disease burden in terms of disability- adjusted life years (DALYs) lost due to disability and premature mortality. In Chile, 93% of new cerebrovascular accidents occur in patients over 45 years of age, resulting in a loss of 70,810 DALYs annually, of which 27,514 are in patients aged 45 to 60 years.

Statistics from the U.S. National Institutes of Health show a progressive increase in hospitalizations for CVA among adolescents and young adults between 1995 and 2008.

Approximately 10% of all cerebrovascular accidents occur in individuals aged 18 to 50 years.

In this patient group, according to the FUTURE study, after an average follow-up of 9 years, 32% had a poor functional outcome, defined as a modified Rankin Scale score >2, meaning they require assistance from others for daily activities.

One of the primary deficits associated with CVA is loss of motor function, particularly upper limb paresis, which is essential for activities of daily living (ADL).

Only 5-20% of patients achieve complete recovery of upper limb function by the sixth month post-CVA, while 30 to 66% of patients will experience partial or incomplete recovery.

Evidence demonstrates that to improve long-term outcomes in terms of ADL, instrumental activities of daily living (IADL), rehospitalization, recurrence, and mortality, neurorehabilitation is essential, delivered through organized, coordinated, and multidisciplinary work by professionals trained in early neurorehabilitation, leading to independence in self-care and potentially successful workforce reintegration.

Regarding the latter point, a variety of rehabilitation therapies exist aimed at restoring upper limb function and independence, as well as patients' social reintegration.

According to studies, achieving proper rehabilitation requires daily rehabilitation sessions over a minimum period of 6 months, where it is observed that 80% of patients reach their peak functional recovery within the first 6 weeks. Therefore, in a patient showing no improvement in the first month, the recovery period will be shortened, and a favorable outcome is not expected. (University of Coruña, 2016)

### MAIN PROBLEMS IDENTIFIED:

- Loss of patient autonomy (ADL). The patient, left with cognitive impairments and motor weakness, requires assistance from a third party for all activities of daily living.
- Loss of body symmetry. Upon suffering the accident, one of the main sequelae the patient may experience is loss of mobility on one entire side of the body, necessitating immediate initiation of a rehabilitation process to regain movement in the affected limb.
- Lack of devices to complement rehabilitation. Currently, there is a shortage of solutions that allow patients to complement rehabilitation autonomously, always requiring third-party assistance for rehabilitation. Available solutions are cumbersome for the patient and highly costly to acquire, both for patients and rehabilitation centers.
- Lack of devices designed for individuals with muscle weakness. Currently, solutions exist to restore body mobility, but they are designed for individuals with some degree of mobility, making them impossible to use for those without mobility in any limb. They require constant third-party assistance.
- Limited remote monitoring by a professional. Due to the scarcity of technological solutions available on the market, remote monitoring of complementary rehabilitation performed by the patient outside rehabilitation centers is complex, preventing the professional from having control or follow-up of session completion, as well as complete lack of knowledge regarding the correct execution of the patient's rehabilitation.
- Delay in starting rehabilitation. Following a cerebrovascular accident, there is high demand to initiate rehabilitation processes, making it impossible for centers to fully meet the need for rehabilitation care. This causes delayed treatment initiation for patients, losing critical recovery time, where only in the first month and a half the greatest probability of recovery can be evidenced, time that is irrecoverable.
- Scarcity of rehabilitation sessions. Rehabilitation centers, lacking the capacity to attend to all incoming patients, can only provide a certain number of rehabilitation sessions. They deliver 2 to 3 sessions per week, which is insufficient for proper recovery. To achieve favorable results, rehabilitation must be performed 7 days a week, 1 to 3 times a day for a minimum period of 6 months.

In the prior art and from the same applicant as the present application, there is application CL201903515 dated December 2, 2019, which defines a kinesic tool for the rehabilitation of individuals with hemiparesis, hemiplegia, and motor weakness, among others. It is formed of a rectangular base, a set of symmetrical rails joined in an H-shape, two parallel transverse rails and one longitudinal to the base, joined together by a rectangular structure that connects them at their ends to the two parallel rails, characterized in that it further comprises a structure that moves between the horizontal rails and serves as support for a grip element, which can be removed and adapted to different possible positions for hands and for feet; additionally, a resistance is attached to the structure that contacts a trajectory restricting the path in a course generating friction upon displacement, and an inclination element is attached to the base.

Additionally, at least two relevant companies in the field are known: Hocoma AG (^{®}) **and Artromot** (^{®}). The former has a system that collects information on completed sessions, requires an arm over the affected limb, its technology consists of a robotic arm plus a computer that requires a desktop to view the exercises to perform. Regarding Artromot, it generates automated movements via a remote control. Similarly, its system is invasive as it requires placing the limb inside a robotic arm for exercise, which is handled by a remote control. In the same way, its structure is similar to a wheelchair with an extension of an automated arm.

Prior art devices, although attempting to perform telerehabilitation, lack devices that meet all requirements for proper rehabilitation and allow follow-up. Rehabilitation centers face the need to require patients to attend rehabilitation gyms, but these are typically high-risk individuals, affected economically by not being able to deliver rehabilitation services due to low permitted occupancy in enclosed and reduced spaces.

Specialists attempt to complement rehabilitation due to lack of resources and implementation by the center to meet this high demand, assigning exercises for home, but these are not performed by the patient for various factors: lack of tools for home, lack of tools or devices designed for individuals with motor weakness preventing the acquisition of additional injuries to those already diagnosed, and that allow performing rehabilitation autonomously, an essential feature since around 30% to 40% of survivors after a CVA are not in condition to return to work and require assistance to perform basic activities of daily living along with their rehabilitation.

In the country, it is observed that approximately 7,954 people annually are losing the possibility of actively reintegrating into society, by being left with a high degree of dependence on others, becoming an economic burden not only for themselves but for their families and the state. Additionally, with the health pandemic, centers are assigning a large part of exercises for home, which cannot be controlled and monitored, where performing them incorrectly could lead to even more cases of disability.

### DESCRIPTION OF THE INVENTION

The solution relates to a playful rehabilitation and monitoring and/or evaluation system, compact, transportable, that can be used on any surface, place and/or environment, which has a touchscreen monitor that through an application projects images, words, patterns to follow, games, etc.

Upper limb motor function is promoted, performing shoulder abduction and external rotation, elbow flexion and extension, among others.

Additionally, through electronic means such as microprocessors and electronic communication devices along with the association of a web platform (World Wide Web) through web applications and software, rehabilitation and progress plans are implemented that help establish realistic goals and manage expectations. Allowing effective communication with a therapist and other health professionals, and also helping patients stay motivated and committed to the rehabilitation process.

The system features various sensors that allow all rehabilitation performed by the user to be stored and subsequently analyzed, enabling the specialist to have control and a record of said rehabilitation. These sensors allow calculating the time the user spends performing sessions, the speed at which the session was executed, the resistance level, the movement dispersion, i.e., with what pressure relative to a pattern it was performed, and the number of repetitions performed per session. All data collection will be key for subsequent analysis, and to obtain in the future a predictive analysis of how a possible rehabilitation could be according to the entered user.

To achieve adherence to rehabilitation sessions, the system has a monitor, which displays the application, and the user can interact with it. To start using the application, it is essential that the user is previously registered and assigned a session of exercises, where the specialist will have to specify the level at which the user will begin their sessions, the number of repetitions per exercise, and the tilt angle (0° to 50°); to assign these specifications, a diagnosis must be performed beforehand so that each session is tailored to the state in which each patient is and as personalized as possible.

Once the assigned users are ready to start their exercise sessions, they must follow the instructions indicated on the monitor: how to log in, verify that the tilt angle is as stipulated by the specialist, where this has sensors that detect the angle at which the device is positioned, and as it is adjusted, it will change and show this change on the monitor, thus knowing it with total precision. Similarly, the level assigned by the specialist and the next exercises to perform will be displayed on the monitor. When the user is ready to start, a virtual assistant will tell them visually and audibly the exercise and the number of repetitions to perform, and "check points" will appear on the screen that must be followed as the user performs the exercise, along with lights displayed on the device that will allow knowing where they are and guiding the movement. Once the exercise is finished, the user will receive a small feedback on the session's performance.

At a cognitive level, the user having this type of rehabilitation, both kinesic and cognitive at the same time, generates greater impact on the evolution of rehabilitation, achieving reduced therapy time.

The system allows automatic data collection from performed sessions. Which aids in the follow-up of patients' sessions by treating specialists and/or caregivers.

Through the configuration of assigned sessions, the software allows the display of dynamic exercises to be performed. Immediate correction can be made to the user in point-to-point reach and tracing of routes (different exercises to be performed) quantifying the movements of the upper limb in the worked area.

An interactive web platform/software, for the control, follow-up, and analysis of the user's rehabilitation, allows specialists to make objective decisions regarding the data provided by the software according to the number of sessions, repetitions, time allocated to each exercise, and accuracy over time in performing the exercises through the display of graphs reflecting the execution of exercises performed by patients, thus enabling periodic reevaluation, allowing measurement of progress toward recovery, measuring speed, dispersion, repetitions, setting sessions, levels, and angles.

It also allows the interactive application from which the user receives feedback regarding their rehabilitation in real time, visually and audibly.

Similarly, under the plate system, there is an LED lighting system, which guides the movement the patient must perform, in addition to turning on lights in different parts of the work areas so that the patient heads to that illuminated direction. In this way, the exercises can help rebuild neural pathways for physical activity and help exercise cognitive and memory functions simultaneously to aid conditions like aphasia, helping improve attention and problem-solving in people who have suffered a brain injury or neurological disorder. All rehabilitation is stored, processed, analyzed, and displayed in the invention's software for subsequent follow-up by the therapist, allowing rehabilitation of multiple patients at once and providing a patient with a therapist- controlled therapy.

A rehabilitation control and monitoring system helps professionals in decision-making by providing real-time data on the patient's evolution and response to treatment. This data can be used to adjust treatment plans, set goals, and track progress over time. Additionally, the system could provide alerts on possible problems or complications, allowing professionals to address them quickly and effectively. By providing accurate and reliable data, the control and monitoring system can improve the overall effectiveness of rehabilitation and enhance patient outcomes.

In the system, different exercise patterns are incorporated, where these can vary from one model to another in order to provide the user with progressively difficult patterns to increase the benefits of rehabilitation, in addition to allowing recovery of body symmetry and improving muscle tone. Additionally, it provides the possibility of performing repetitive movements; repeating exercises helps rebuild neural pathways involved in muscle control so that the patient can eventually improve in practical tasks like dressing, eating, or writing.

Additionally, it has a glove that allows attachment to a grip system in case the user lacks grip strength to hold on, and mobility to perform the exercises; in this case, the user would have to perform sessions with both limbs where the limb with mobility guides the movement to the other, where in cognitive rehabilitation it would be very similar to what occurs with mirror therapy. Additionally, thanks to this glove, the user can perform all their rehabilitation session autonomously, without needing to rely on third-party assistance.

The system comprises a compact, transportable device that can be used on any surface, place, and/or environment, with guide means on the upper surface of the device, allowing restriction of movement in case the user is in a very early stage of their rehabilitation and has little or no movement of their upper limb, where performing uncontrolled or free movement could cause some type of additional muscular sequela (movement restriction). This guide attaches to different exercise plates with a groove that allows the guide to be inserted within without coming out of the path to follow, providing security to the user in their rehabilitation. The plates contain different types of paths which are symmetrical and functional, and help in recovering body symmetry lost due to some motor damage in a limb.

The guide protruding from the grip/handle system connects to these paths to instantly achieve restriction of movements, and the user, by mobilizing their upper limb, is not allowed to deviate from the pattern obtaining a controlled, safe rehabilitation, thus avoiding involuntary movements that could cause additional injuries due to a lack of sufficient muscle, where the shoulder joint could deform (Subluxation).

### DESCRIPTION OF THE FIGURES

In order to better understand the present invention and appreciate its practical applications, the following figures are provided, to which reference is made below. It should be noted that the figures are given only as examples, and in no way limit the scope of the invention. The same components are indicated with the same reference numbers, in which:
Figure 1 Shows an isometric view of the device of the invention.
Figure 2 Shows a top isometric view with the assisted path plate.
Figure 3 Shows a top isometric view of the device of the invention in its stored state.
Figure 4 Shows an inclined isometric view of the device of the invention illustrating the deployment means.

### DETAILED DESCRIPTION OF THE INVENTION

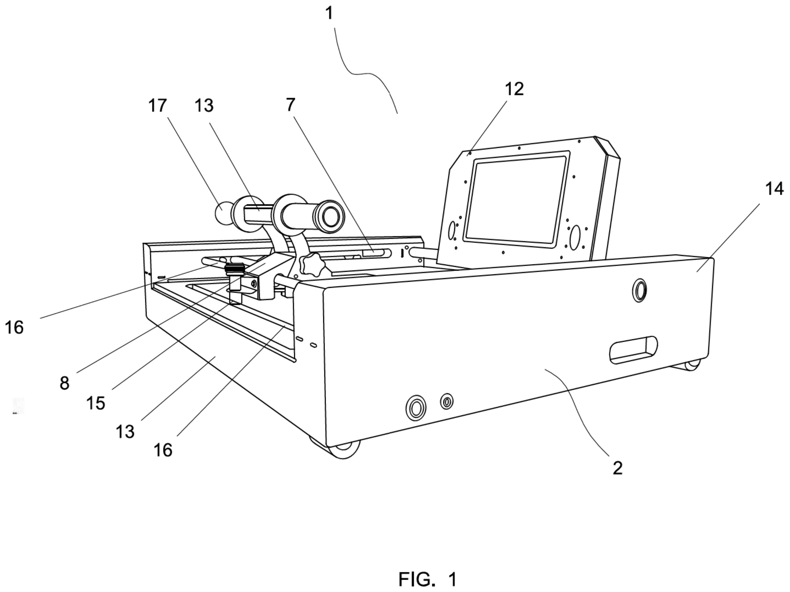

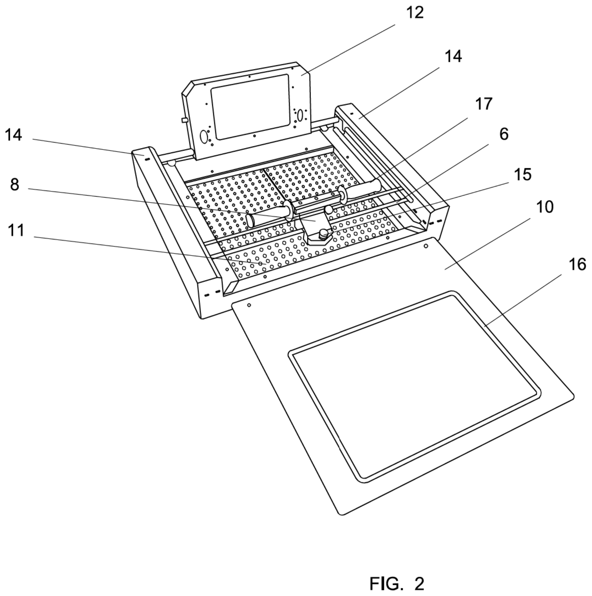

With reference to figures 1 and 2, it is shown that the technological rehabilitation and monitoring compact system that allows promoting motor function, comprises a device (1) to be used by a seated or standing person, which has sufficient size to exercise the upper body and practice exercise movements of a range of motion for the joints, consists of a chassis (2) comprising a base (3) of generally rectangular shape, with two wings (14) protruding upward from the base, defining a double L shape in horizontal position. The device has symmetrical rails joined in an H shape, two parallel transverse rails (6) mounted slidably in a cavity in each wing (14) and two longitudinal rails (7) one in each wing (14) at a predefined distance from the surface of the chassis base and with means of joining each other, so that the transverse rails slide on the longitudinal rails.

Between the transverse rails, a rectangular structure (8) is also defined on a surface of the base (3), joined slidably to the transverse rails (6), the rectangular structure (8) serves as support for a grip system (13), which can be adapted to different positions for hands, additionally a restrictive guide (15) is attached to the structure that touches directly a path groove (16) generating friction upon displacement.

The rail system allows mobilizing the arm and shoulder in a full range of movements, such as shoulder abduction and external rotation, elbow flexion and extension, among others. In this way, it allows the user to improve the range of motion by providing resistance and assistance to the affected joint or body part, which allows moving in a full range of motion. This can help reduce stiffness and pain, as well as increase flexibility and mobility. Reduce pain and stiffness, improve coordination and fine motor skills, improve overall function and independence in daily activities, and improve quality of life in general.

The grip system (13) is adjustable in different positions allowing it to be used with one or both hands, depending on the patient's injury, it is elastic and adaptable in different positions, vertical and horizontal for both hands, and vertical only for one hand, depending on the weakness and need of the user to be treated and if they do not have grip strength, a special kinesic glove adaptable to the handles (17) of the grip system can be used, which allows fixing the hand to the grip system, being this adaptable for the right or left hand of the patient. The grip system is ergonomic and can help improve the comfort and performance of a device, as well as reduce the risk of injuries, during short or long periods of time, reducing fatigue and hand strain.

A plate with interchangeable designs (10) is defined, installed removably on the surface of the chassis base and under the rails; for the restriction of movements in a specific way, and in a particular direction, allowing more or less movement. The different designs restrict movement in different ways, such as limiting rotation, flexion or extension, which allow controlling the movement of the grip system (13) when used by users, by means of the restrictive guide (15) that fits in a path groove (16) in the plate with interchangeable designs (10), which also allows minimizing the risk of re-injury.

To achieve adherence to rehabilitation sessions, the system has a touchscreen monitor (12) adjustable with a tilt of 0° to 180°, foldably joined over the chassis (2), installed on an articulated bar at the rear end of the chassis, which allows folding the touchscreen monitor for storage or transport of the device, which displays a software application with predetermined exercises, which allows interacting with the user visually and audibly, allowing visualization of the exercises and playful rehabilitation, also delivering feedback of the user's performance after each exercise session.

For auditory interaction, a sound system mounted inside the chassis is defined, i.e., speakers and amplifiers integrated into the chassis, which has a physical knob or dial also installed in the chassis to adjust the volume, which can be turned clockwise or counterclockwise to increase or decrease the volume.

With reference to figure 2, the **LED** panel (11) installed under the plates with interchangeable designs (10) is observed, which shows the movement to be performed or where to go, useful for guiding users through a task or activity by providing clear and easy-to-follow visual signals.

The LED panel (11) has a light system that indicates progress and/or guides the path over a surface, to stimulate and guide the user, allows performing free exercises being able to have mobility in it in all directions and forms.

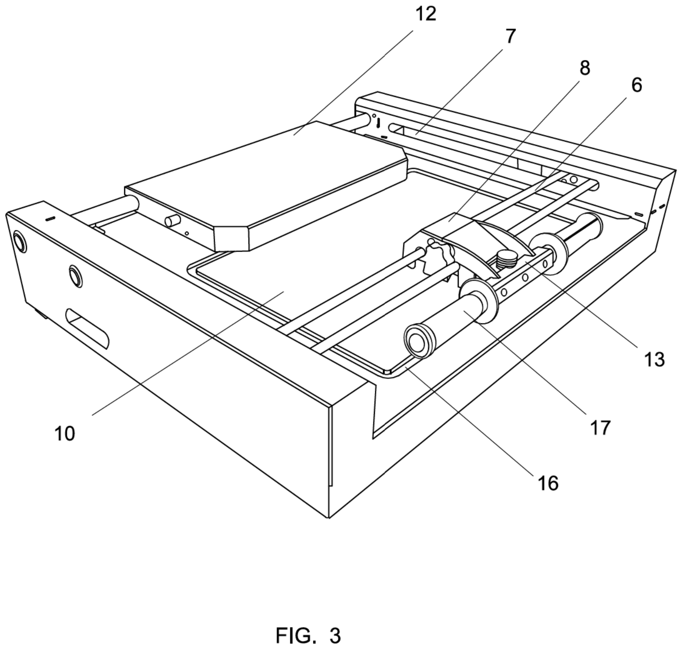

In figure 3, the system is illustrated in a folded position with the touchscreen monitor (12) folded and the grip system in rest position so that easy, comfortable transport and storage is allowed.

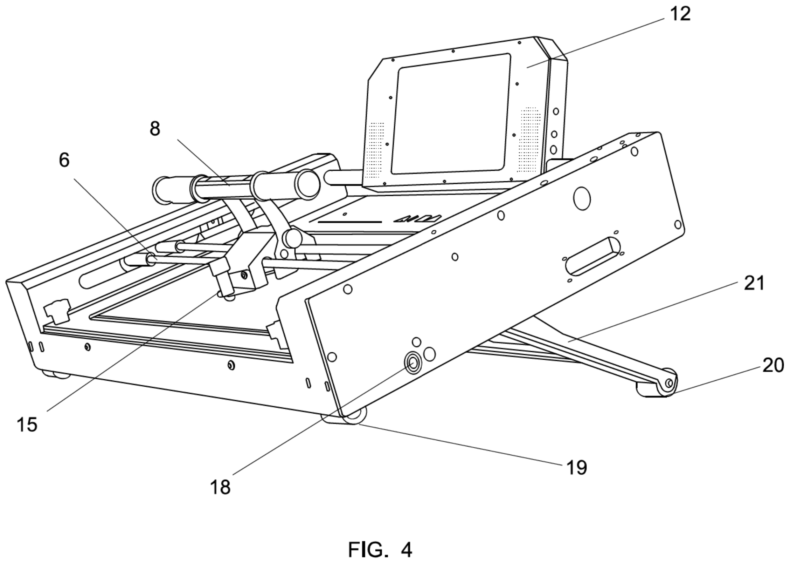

In figure 4, it is illustrated that the rectangular chassis (2) connects to a pin and button release system (18) of a support arm (20), which allows the inclination of the device in different degrees of freedom due to the articulated support arm (20); also, front wheels (19) are defined at the lower front part of the chassis, and a rear wheel (20) at the end of the support arm, which allow the chassis to be accommodated on a support surface to suit the user's needs. By means of the support arm (20) the device can be tilted with different angles of inclination, from 0° to 50°, for the exercise of the upper body and also which guarantee the user to work different types of muscles and add difficulty in their usability, in addition to making rehabilitation more complex at a higher angle of inclination, due to the force of gravity and the precision needed to perform the sessions.

The rehabilitation and monitoring system incorporates sensors inserted in the chassis (2) under the plate with interchangeable designs (10) and the LED panel (11), for the detection, collection, and monitoring of the patient's rehabilitation, detect the tilt angles at which the device is positioned, the rehabilitation time, the position and dispersion of movements, the number of repetitions, among other measurements. They allow recording the movements performed by the user to prepare statistics such as usage time, number of repetitions, speed, precision, and resistance level.

The embodiment of the described application is intended to be only an example. Experts in this technology will understand that various modifications of details to this embodiment can be made, all of which are within the scope of the application.

## Claims

1. Rehabilitation and monitoring and/or evaluation system, compact, transportable, that can be used on any surface, place and/or environment, to promote motor function, to be used by a seated or standing person, which has sufficient size to exercise the upper body and practice range of motion exercises for the joints, consisting of a chassis with a generally rectangular base, a grip system for the user's hands over the surface of the base with symmetrical rails mounted on the chassis, joined in an H shape, two parallel transverse rails slidably joined to two longitudinal rails, so that the transverse rails slide on the longitudinal rails, so that the user through the grip system moves their arms and hands in a controlled manner over the surface of the base, whether the chassis is in horizontal position or with some degree of inclination, CHARACTERIZED furthermore, because:
- the chassis defines two wings protruding upward from the base, defining a double L shape in horizontal position, with the transverse rails and the two longitudinal rails mounted in a cavity in each wing at a predefined distance from the surface of the chassis base;
- the grip system is adjustable in different positions to be used with one or both hands, horizontal for both hands, and vertical for one hand;
- includes a kinesic glove adaptable to handles of the grip system, which allows fixing the hand to the grip system;
- a pin and button release fixation system of an articulated support arm that allows the inclination of the chassis in different degrees of freedom, from 0 to 50°;
- plates with interchangeable designs for the restriction of movements of the grip system, installed removably over the surface of the chassis base and under the rails;
- an LED panel under the plates with interchangeable designs, which shows the movement to be performed or where to go, useful for guiding users through a task or activity by providing clear and easy-to-follow visual signals;
- a light system in the LED panel that indicates progress and/or guides the path over the surface of the panel;
- a sound system mounted inside the chassis, with a physical knob or dial to adjust its volume;
- a sensor system inserted in the chassis under the LED plates and plate with interchangeable designs;
- an adjustable touchscreen monitor with a tilt of 0° to 180°, over the chassis and installed on an articulated bar at the rear end of the chassis that allows interacting with the user visually and audibly; and
- electronic means such as microprocessors and electronic communication devices that are associated with the sensors, a web platform (World Wide Web), a web application and software, for the control, follow-up, and analysis of the user's rehabilitation, allowing interactivity with specialists, for the follow-up and analysis of the user's rehabilitation.

2. Rehabilitation and monitoring and/or evaluation system, compact, transportable, that can be used on any surface, place and/or environment, according to claim 1, CHARACTERIZED furthermore because: the web platform/software, allows specialists to make objective decisions regarding the data provided by the software according to the number of sessions, repetitions, time allocated to each exercise, and accuracy over time in performing the exercises through the display of graphs reflecting the execution of exercises performed by patients, thus enabling periodic reevaluation, allowing measurement of progress toward recovery, measuring speed, dispersion, repetitions, setting sessions, levels, and angles.

3. Rehabilitation and monitoring and/or evaluation system, compact, transportable, that can be used on any surface, place and/or environment, according to claim 1, CHARACTERIZED because furthermore comprises: a rectangular structure slidably joined to the transverse rails, and serving as support for the grip system, which can be adapted to different hand positions, additionally to the rectangular structure, a restrictive guide is attached that protrudes from the grip system and touches directly a path groove in the plates with interchangeable designs generating friction upon displacement.

4. Rehabilitation and monitoring and/or evaluation system, compact, transportable, that can be used on any surface, place and/or environment, according to claim 3, CHARACTERIZED furthermore because the plates with interchangeable designs contain different types of paths which are symmetrical and functional, and help in recovering body symmetry lost due to some motor damage in a limb.

5. Rehabilitation and monitoring and/or evaluation system, compact, transportable, that can be used on any surface, place and/or environment, according to claim 1, CHARACTERIZED furthermore because the LED lighting system, which guides the movement the patient must perform, comprises lights in different parts of the work area in the LED panel so that the patient heads to that direction when illuminated.

6. Rehabilitation and monitoring and/or evaluation system, compact, transportable, that can be used on any surface, place and/or environment, according to claim 1, CHARACTERIZED furthermore because the adjustable touchscreen monitor with a tilt of 0° to 180°, is foldably joined to the chassis for its storage or transport, which displays a software application with predetermined exercises, which allows interacting with the user visually and audibly, allowing visualization of the exercises and playful rehabilitation, also delivering feedback of the user's performance after each exercise session.

7. Rehabilitation and monitoring and/or evaluation system, compact, transportable, that can be used on any surface, place and/or environment, according to claim 1, CHARACTERIZED furthermore because the sensors that are inserted in the chassis under the plate with interchangeable designs and LED panel, detect tilt angles at which the chassis is positioned, the rehabilitation time, the position and dispersion of movements, the number of repetitions, and detect the movements performed by the user, which allows preparing statistics such as usage time, numberof repetitions, speed, precision, and resistance level.

8. Rehabilitation and monitoring and/or evaluation system, compact, transportable, that can be used on any surface, place and/or environment, according to claim 1, CHARACTERIZED furthermore because the plates with interchangeable designs, have different designs of the grooves' paths, to restrict movement in a specific way, in a particular direction, allowing more or less movement, such as limiting rotation, flexion or extension.

9. Rehabilitation and monitoring and/or evaluation system, compact, transportable, that can be used on any surface, place and/or environment, according to claim 1, CHARACTERIZED furthermore because the web application is interactive and allows the user to receive feedback regarding their rehabilitation in real time, visually and audibly.
